# EUROPÄISCHE PATENTANMELDUNG

(11) **EP 1 097 693 A2**
(43) Veröffentlichungstag der Anmeldung: **09.05.2001**
(21) Anmeldenummer: 00120794.3
(22) Anmeldetag: 23.09.2000
(51) Int. Cl.: A61K 7/00, A61K 7/32

(54) **Mikrokapselsysteme zur umweltinduzierten Abgabe von Wirkstoffen**

(30) Priorität: 02.11.1999 DE 19952586
(71) Anmelder: Deotexis Inc., New York, N.Y. 10022-4838 (US)
(72) Erfinder: Tebbe, Gerold, 98000 Monte Carlo (MC)
(74) Vertreter: Ostertag, Reinhard

(57) **Zusammenfassung**

Ein Mikrokapselsystem (10) wird dazu eingesetzt, Wirkstoffe (W1, W2) zu speichern und abhängig von bestimmten Umweltparametern abzugeben. Die Wirkstoffe (W1, W2) sind dabei in Kapseln (12, 14) mit einer Kapselwand (16, 18) enthalten, die ein umweltinduziertes Material umfaßt. Um eine sequentielle zeitgesteuerte Abgabe der Wirkstoffe (W1, W2) zu ermöglichen, ist vorgesehen, daß eine Mehrzahl von Kapseln (14) in mindestens einer äußeren Kapsel (12) aufgenommen ist und so eine innere Kapselungsstufe bildet, wobei die äußeren Kapseln (12) eine äußere Kapselungsstufe bilden. Die Kapselwand (16) der äußeren Kapsel (12) ist ebenso wie die Kapselwand (18) der inneren Kapseln (14) aus einem umweltinduzierten Material.

## Beschreibung

Die vorliegende Erfindung betrifft ein Mikrokapselsystem zur umweltinduzierten Abgabe von Wirkstoffen gemäß dem Oberbegriff des Anspruchs 1.

Dabei wird hier und nachfolgend unter "umweltinduziert" verstanden, daß die Kapselwände der Kapseln des Mikrokapselsystems ein Material umfassen, welches unter dem Einfluß eines oder mehrerer bestimmter Umweltparameter instabil wird, so daß die entsprechenden Kapseln durchlässig werden oder aufplatzen und den ggf. in ihnen enthaltenen Wirkstoff freigeben. Beispiele für entsprechende Umweltparameter sind z.B. Druck, Temperatur, Verformung, Licht und andere elektromagnetische Strahlung, partikuläre Strahlung, chemische Substanzen, etc. Auch die Zeit soll für die Zwecke der Erfindung als Umweltparameter angesehen werden.

Ein solches Mikrokapselsystem ist z.B. aus der DE 40 00 920 A1 bekannt. Dort sind mit Pflegeöl gefüllte Mikrokapseln auf der Oberfläche eines Tuches aufgebracht. Auf diese Weise ist das Pflegeöl während der Lagerzeit des Tuchs vor Verdunsten geschützt. Wird das Tuch z.B. auf der Haut gerieben, zerplatzen aufgrund der Verformung die Kapselwände, so daß das in den Kapseln enthaltende Pflegeöl an die Umgebung freigesetzt wird und seine Wirkung auf der Haut des Benutzers entfalten kann.

In manchen Anwendungsfällen kann es jedoch wünschenswert sein, daß nicht alle Wirkstoffe auf einmal freigesetzt werden, sondern daß die Freigabe der Wirkstoffe aus den jeweiligen Mikrokapseln in einer bestimmten zeitlichen Abfolge erfolgt.

Diese Aufgabe wird durch ein Mikrokapselsystem mit den im Anspruch 1 angegebenen Merkmalen gelöst.

Durch die erfindungsgemäß vorgesehene "Verschachtelung" von inneren Kapseln in mindestens einer äußeren Kapsel ist es möglich, ein sequentielles, also in zeitlichen Stufen erfolgendes Freigeben der Wirkstoffe zu erreichen. Die in dem Mikrokapselsystem gespeicherten Wirkstoffe werden also nicht unbedingt auf einmal aus den jeweiligen Mikrokapseln freigegeben, sondern in einer frei wählbaren zeitlichen Abfolge. Hierdurch erschließen sich für das erfindungsgemäße Mikrokapselsystem zahlreiche neue und auf die jeweiligen Anforderungen präzise abstimmbare Anwendungsmöglichkeiten.

Vorteilhafte Weiterbildungen der Erfindung sind in Unteransprüchen angegeben.

Durch die Weiterbildung der Erfindung gemäß Anspruch 2 wird die Anzahl der möglichen zeitlichen Stufen erhöht. Eine Grenze für die Anzahl der möglichen Kapselungsstufen ergibt sich nur aus der maximal möglichen Größe der Kapseln der äußersten Kapselungsstufe, welche von einer Anwendung zur anderen variieren kann.

Gemäß Anspruch 3 ist es möglich, beim Aufbrechen einer Kapselungsstufe gleichzeitig relativ große Wirkstoffmengen aus dieser Kapselungsstufe freizusetzen.

Komplexe Freigabesequenzen der in den Kapseln einer Kapselungsstufe enthaltenen Wirkstoffe sind durch die beiden Weiterbildungen der Erfindung gemäß Anspruch 4 und Anspruch 5 realisierbar.

In vielen Fällen wird es wünschenswert sein, nicht den gleichen, sondern unterschiedliche Wirkstoffe sequentiell freizugeben. Dies ist in Anspruch 6 angegeben.

Gemäß Anspruch 7 erfolgt das Aufbrechen der Kapselungsstufen von außen nach innen. Die Wirkstoffe werden also nicht nur aus den entsprechenden Kapseln sequentiell freigesetzt sondern auch seqentiell an die Umgebung abgegeben.

Bei der Weiterbildung der Erfindung gemäß Anspruch 8 erfolgt das Aufbrechen dagegen in der umgekehrten Richtung, d.h. also von innen nach außen. Dies ist vor allem dann von Vorteil, wenn z.B. unterschiedliche Wirkstoffe zunächst miteinander vermischt werden sollen, bevor die äußerste Kapselungsstufe aufbricht und die so erzeugte Wirkstoffmischung an die Umgebung abgegeben wird.

Nachstehend wird die Erfindung anhand von zwei Ausführungsbeispielen unter Bezugnahme auf die Zeichnung näher erläutert. In dieser zeigen
- Figur 1:: einen stark vergrößerten Schnitt durch ein erstes Ausführungsbeispiel eines Mikrokapselsystems;
- Figur 2:: eine Ansicht ähnlich Figur 1 nach dem Aufplatzen der Mikrokapseln einer äußeren Kapselungsstufe des ersten Ausführungsbeispiels;
- Figur 3:: eine Ansicht ähnlich Figur 2 nach dem Aufplatzen der Mikrokapseln einer inneren Kapselungsstufe des ersten Ausführungsbeispiels;
- Figur 4:: einen stark vergrößerten Schnitt durch ein zweites Ausführungsbeispiel eines Mikrokapselsystems;
- Figur 5:: eine Ansicht ähnlich Figur 4 nach dem Aufplatzen der Mikrokapseln einer inneren Kapselungsstufe des zweiten Ausführungsbeispiels;
- Figur 6:: eine Ansicht ähnlich Figur 5 nach dem Aufplatzen der Mikrokapseln einer mittleren Kapselungsstufe des zweiten Ausführungsbeispiels;
- Figur 7:: eine Ansicht ähnlich Figur 6 nach dem Aufplatzen der Mikrokapseln aller mittleren Kapselungsstufen des zweiten Ausführungsbeispiels;
- Figur 8:: eine Ansicht ähnlich Figur 7 nach dem Aufplatzen der Mikrokapseln einer weiteren inneren Kapselungsstufe des zweiten Ausführungsbeispiels;
- Figur 9:: eine Ansicht ähnlich Figur 8 nach dem Aufplatzen der Mikrokapseln der äußeren Kapselungsstufe des zweiten Ausführungsbeispiels; und
- Figur 10:: einen Schnitt durch ein Anwendungsbeispiel des Mikrokapselsystems der Figuren 4 bis 9.

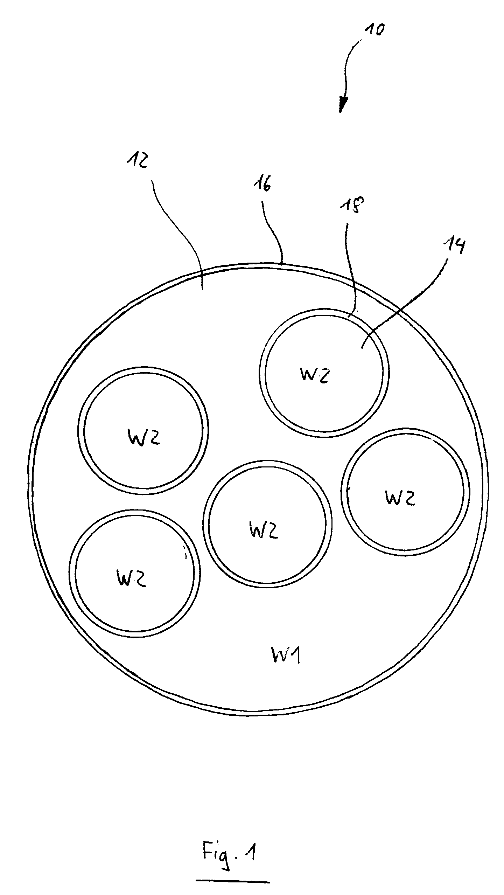

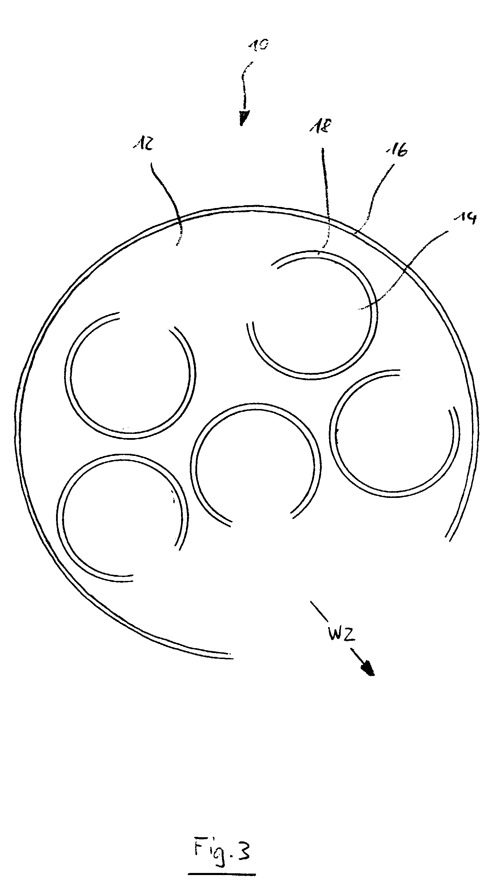

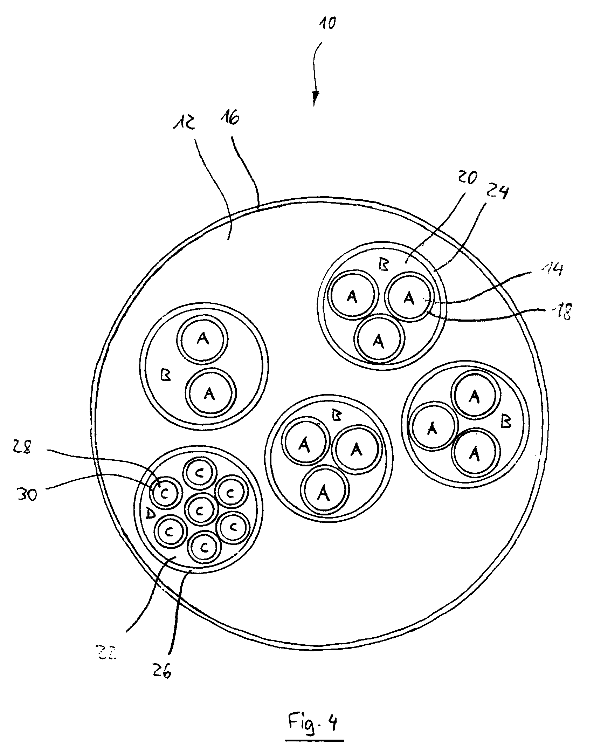

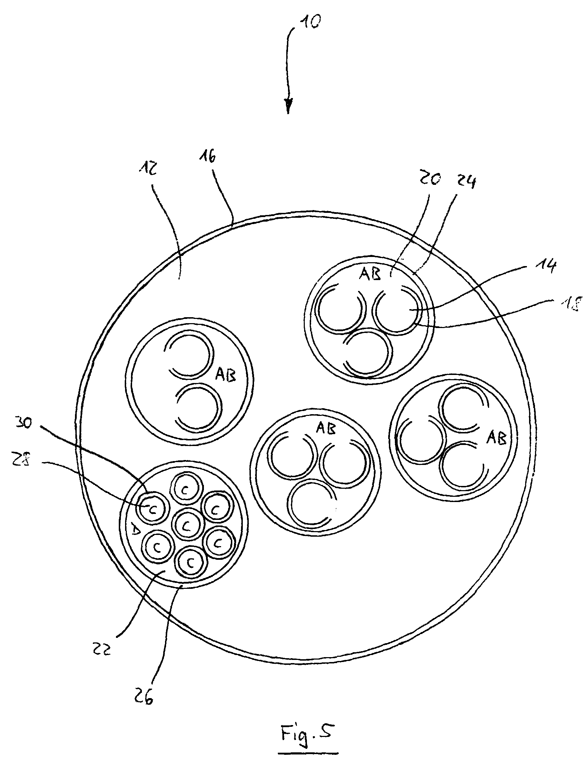

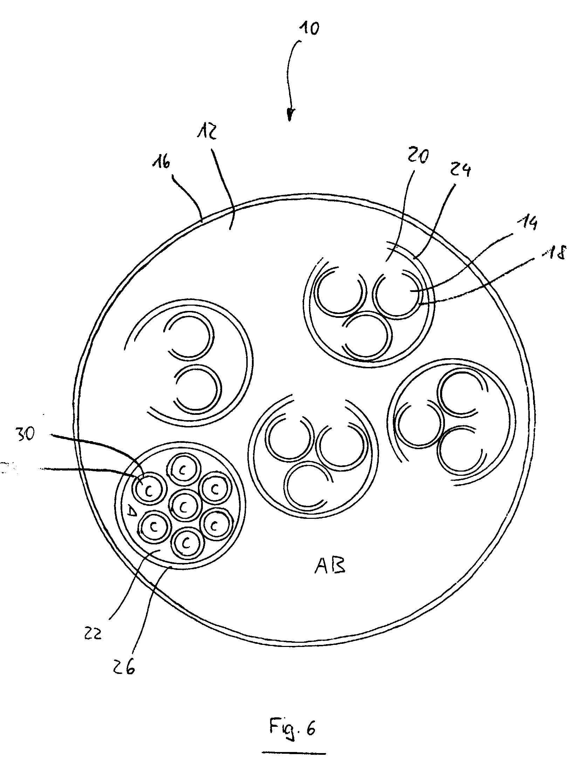

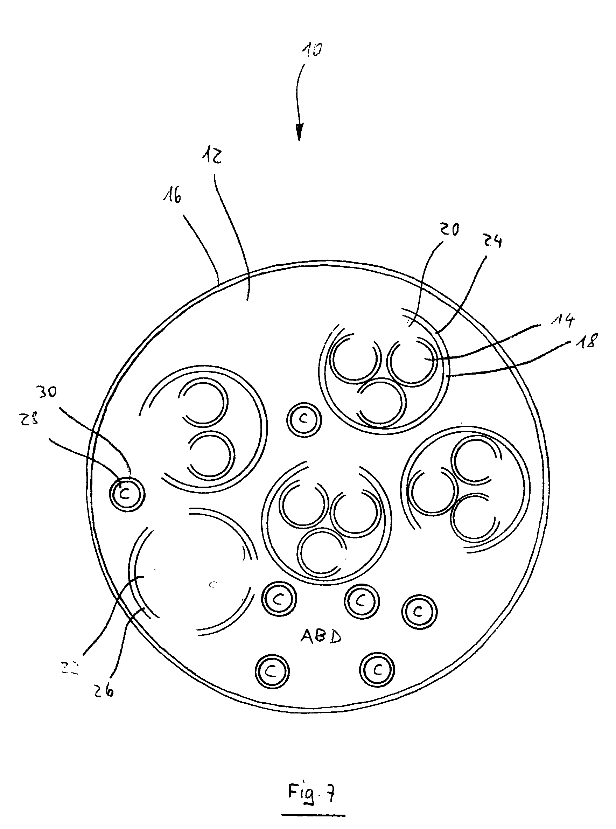

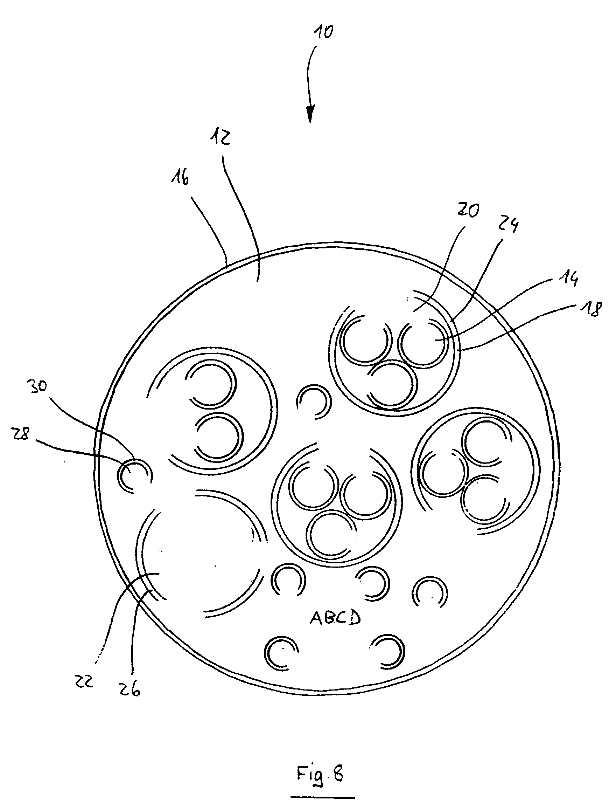

In Figur 1 trägt ein Mikrokapselsystem insgesamt das Bezugszeichen 10. Es umfaßt eine Vielzahl von äußeren Kapseln 12 einer äußeren Kapselungsstufe, von denen in den Figuren 1 bis 3 jedoch nur eine dargestellt ist. Diese wird nachfolgend der Einfachheit halber als äußere Kapsel 12 bezeichnet.

Die äußere Kapsel 12 enthält einen Wirkstoff W1 sowie eine Mehrzahl von Kapseln 14 einer inneren Kapselungsstufe. Diese enthalten einen Wirkstoff W2 und werden nachfolgend innere Kapseln 14 genannt.

Die Kapseln 12 und 14 sind jeweils von einer sehr dünnen Kapseiwand 16 bzw. 18 umgeben, die ein umweltinduziertes Material umfaßt. Bei dem vorliegenden Ausführungsbeispiel bedeutet dies, daß die äußere Kapselwand 16 unter dem über einen gewissen Zeitraum andauernden Einfluß einer Temperatur in Höhe der menschlichen Körpertemperatur aufplatzt, wohingegen das Material der Kapselwand 18 der inneren Kapseln 14 ein Material umfaßt, welches sich unter der länger andauernden Einwirkung von menschlichem Schweiß zersetzt und so die Kapseln 14 aufplatzen läßt.

Beim vorliegenden Ausführungsbeispiel handelt es sich bei dem Wirkstoff W1 um eine relativ milde chemische Substanz, welche mit dem menschlichen Schweiß reagiert, um diesen zu neutralisieren. Beim Wirkstoff W2 handelt es sich um ähnliche Substanz, die jedoch höher konzentriert ist und somit eine höhere Wirksamkeit aufweist.

Das Mikrokapselsystem 10 kann entweder auf die in der DE 40 00 920 A1 beschriebene Art und Weise mit einem Bindemittel auf ein Tuch aufgebracht werden. Alternativ kann das Mikrokapselsystem 10 auch in einer Emulsion aufgenommen sein, die direkt auf die entsprechenden Stellen auf der menschlichen Haut aufgetragen wird.

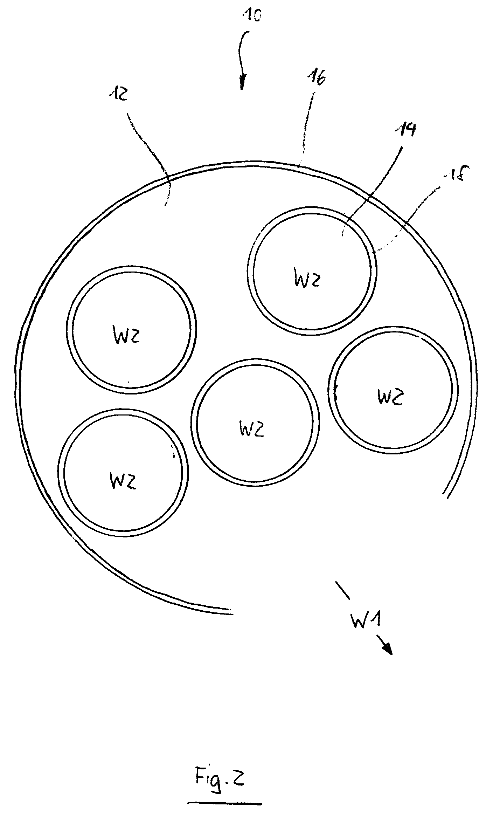

Wie aus Figur 2 ersichtlich ist, platzt unter dem Einfluß der Körpertemperatur zunächst die Kapselwand 16 der äußeren Kapsel 12 auf, wodurch der Wirkstoff W1 sowie die inneren Kapseln 14 freigesetzt werden. Der Wirkstoff W1 gelang somit direkt auf die Haut des Benutzers und kann die Zersetzung dort vorhandenen Schweißes verhindern und weiterer Schweißbildung entgegenwirken.

Wird jedoch z.B. bei einer länger andauernden starken körperlichen Betätigung eine große Schweißmenge abgesondert, welcher vom Wirkstoff W1 nicht mehr in ausreichendem Maße begegnet werden kann, zersetzt sich unter der Einwirkung dieses Mehrs an Schweiß die Kapselwand 18 der inneren Kapseln 14, wodurch der erhöht wirksame Wirkstoff W2 freigesetzt wird. Auf diese Weise ist eine bedarfsorientierte sequentielle Abgabe unterschiedlich wirksamer Wirkstoffe W1 und W2 möglich.

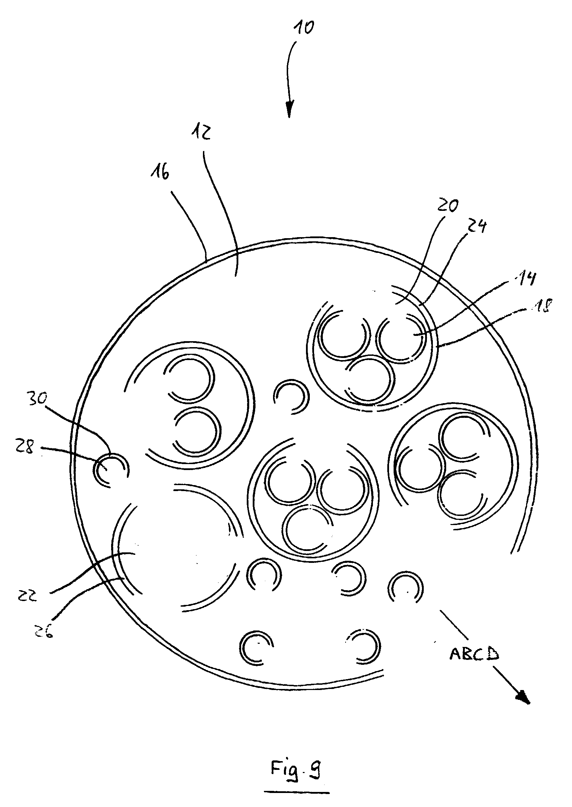

Nun wird auf das in den Figuren 4 bis 9 dargestellte zweite Ausführungsbeispiel eines Mikrokapselsystems 10 Bezug genommen. Funktionsäquivalente Teile sind darin mit den gleichen Bezugszeichen versehen wie in dem in den Figuren 1 bis 3 dargestellten Ausführungsbeispiel. Im übrigen wird nachfolgend vor allem auf die Unterschiede zum ersten Ausführungsbeispiel eingegangen.

Das in den Figuren 4 bis 9 dargestellte Mikrokapselsystem 10 ist dreistufig aufgebaut und dient zur Herstellung einer Schicht, welche einen durch Druck aktivierbaren Mehrkomponentenkleber umfaßt.

Das Mikrokapselsystem 10 umfaßt wiederum eine Mehrzahl von Kapseln 12 einer äußeren Kapselungsstufe, welche eine dünne und relativ poröse, gasdurchlässige Kapselwand 16 aufweisen. In den Figuren 4 bis 8 ist nur eine der Kapseln 12 der äußeren Kapselungsstufe dargestellt. Der Einfachheit halber wird diese nachfolgend als äußere Kapsel 12 bezeichnet.

Diese äußere Kapsel 12 umschließt eine Mehrzahl von Kapseln 20 und 22 einer mittleren Kapselungsstufe, die jeweils von einer dünnen Kapseiwand 24 bzw. 26 umgeben sind und nachfolgend als mittlere Kapseln 20 und 22 bezeichnet werden. Die mittleren Kapseln 20 enthalten wiederum einen Wirkstoff B, vorliegend Mikroballons, die beim fertigen Klebstoff als Füllstoff dienen, der z.B. beim Auffüllen von Unebenheiten an den Klebeflächen nützlich ist, sowie Kapseln 14 einer inneren Kapse Kapselwand 18, in denen ein Wirkstoff, nämlich Epoxidharz A, enthalten ist. Die Kapseln 14 der inneren Kapselungsstufe werden nachfolgend als innere Kapseln 14 bezeichnet. Die mittleren Kapseln 22 enthalten als Wirkstoff D einen Aushärtungsbeschleuniger sowie Kapseln 28 einer weiteren inneren Kapselungsstufe, welche als Wirkstoff C eine Härtersubstanz enthalten und eine dünne Kapselwand 30 umfassen.

Die Materialien der Kapselwände 16, 18, 24, 26 und 30 sind in folgender Weise umweltinduziert: Die Kapselwand 16 der äußeren Kapsel 12 zersetzt sich bei thermischer Beaufschlagung, genauer bei einer Temperatur oberhalb 40°C. Die Kapselwände 24, 26 und 30 der mittleren Kapseln 20 und 22 bzw. der inneren Kapseln 28 zersetzen sich unter dem Einfluß des Wirkstoffgemisches AB, also des Epoxidharz-Mikroballongemisches. Die Kapselwände 18 der inneren Kapseln 14 platzen unter mechanischer Beaufschlagung, also z.B. unter Druck oder durch Verformung.

Die Funktion des in den Figuren 4 bis 9 dargestellten Mikrokapselsystems 10 wird nun unter Hinzuziehung des in Figur 10 dargestellten Anwendungsbeispiels erläutert.

Zunächst werden die äußeren Mikrokapseln 12 des Mikrokapselsystems 10 mit einer Bindemittelschicht 32 an einer entsprechenden Oberfläche 34 eines Werkstücks 36 aufgebracht. Dann werden die Mikrokapseln 12 von einer weichen, stoßabweisenden Folie 38 abgedeckt. In diesem Zustand kann das Werkstück mit dem Mikrokapselsystem 10 über einen längeren Zeitraum gelagert werden, ohne daß die Klebeschicht aktiv ist oder die einzelnen Komponenten, aus denen die Klebeschicht hergestellt werden wird, sich zersetzen.

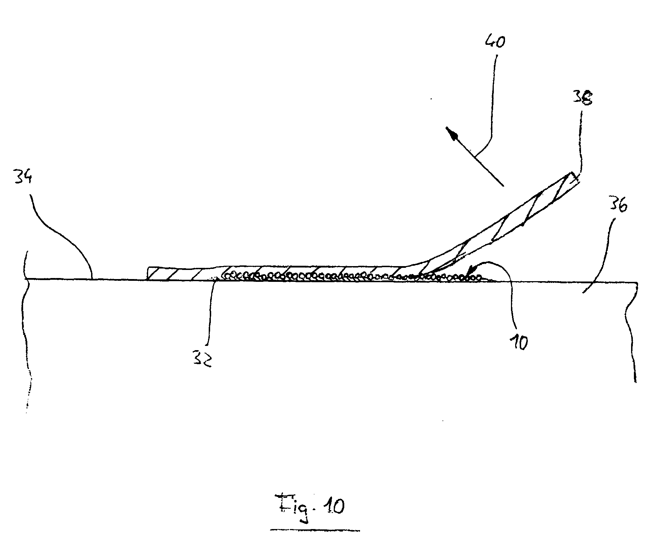

Zur Aktivierung der Klebeschicht, d.h. zum Auslösen der Freigabesequenz der in den Mikrokapseln 12, 16, 20, 22 und 28 enthaltenen Wirkstoffe A, B, C und D, wird die Folie 38 abgezogen, wie dies in Figur 10 durch den Pfeil 40 angedeutet ist. Dann wird das Werkstück 36 mit der aus dem Mikrokapselsystem 10 bestehenden Schicht gegen die Wand gedrückt, auf die es aufgeklebt werden soll. Hierdurch werden die Kapselwände 18 der das Epoxidharz A enthaltenden inneren Kapseln 14 verformt, wodurch diese zerplatzen und das Expoxidharz A freigeben. Somit vermischen sich innerhalb der mittleren Kapseln 20 die Mikroballons B und das Epoxidharz A. Dies ist in Figur 5 dargestellt.

Da die Kapselwände 24 der mittleren Kapseln 20 gegenüber dem Epoxidharz A instabil sind, gehen auch diese auf, so daß das Gemisch aus Mikroballons B und Epoxidharz A in den Innenraum der äußeren Kapsel 12 gelangt (vergleiche Figur 6).

Jetzt kommt auch die Kapselwand 26 der mittleren Kapsel 22 mit dem Epoxidharz A in Berührung, wodurch sich diese auflöst und sich innerhalb der äußeren Kapseln 12 neben dem Mikroballon-Epoxidharzgemisch AB auch noch der Aushärtungsbeschleuniger D und die Kapseln 28 mit der Härtersubstanz C verteilen (vergleiche Figur 7).

Da deren Kapselwand 30 jedoch ebenfalls gegenüber dem Mikroballon-Epoxidharzgemisch AB instabil ist, löst sich diese auf, so daß schließlich innerhalb der äußeren Kapsel 12 die vier Substanzen Mikroballons B, Epoxidharz A, Härtersubstanz C und Aushärtungsbeschleuniger D miteinander gemischt vorliegen (vergleiche Figur 8).

Hierdurch setzt eine bei der betrachteten Komponentenwahl exotherme Reaktion ein, welche zu einer Temperaturerhöhung führt, die die Kapseiwand 16 der äußeren Kapseln 12 öffnet, so daß der nun fertig gemischte Mehrkomponenten-Epoxidharzkleber als reagierende und abbindende durchgehende Klebeschicht vorliegt (vergleiche Figur 9).

Durch ein solchermaßen gestaltetes Mikrokapselsystem 10 kann also ein hochfester Mehrkomponentenkleber realisiert werden, dessen einzelne Komponenten zunächst getrennt voneinander vorliegen und daher auch über einen längeren Zeitraum gelagert werden können. Durch einfaches Aufbringen einer Druckbelastung wird die Freigabesequenz des Mikrokapselsystems 10 ausgelöst, wodurch die einzelnen Komponenten des Mehrkomponentenklebers miteinander vermischt werden und dieser aktiviert wird.

Die vorliegende mehrstufige Mikroverkapselung kann selbstverständlich auch in anderen Bereichen Verwendung finden. Hierzu gehören z.B. die sequentielle Abgabe von Duftstoffen, von Medikamenten, von Schmiermitteln oder auch von Dünge- oder Schädlingsbekämpfungsmitteln.

Obenstehend ist von umweltreaktiv aufplatzenden Kapselwänden gesprochen. Es versteht sich, daß auch ein sanfteres und unvollständiges Öffnen der Kapsel brauchbar ist. Wichtig ist nur, daß sich die Durchlässigkeit einer Kapselwand für den Kapselinhalt bei Einwirkung eines Umweltparameters ändert.

## Patentansprüche

1. Mikrokapselsystem zur umweltinduzierten Abgabe von Wirkstoffen, welches eine Mehrzahl von Kapseln (12, 14; 20, 22, 28) mit einer Kapselwand (16, 18; 24, 26, 30) aufweist, die ein umweltinduziertes Material umfaßt, wobei in mindestens einem Teil der Kapseln (12, 14; 20, 22, 28) ein Wirkstoff (W1, W2; A, B, C, D) enthalten ist,
dadurch gekennzeichnet, daß
die Mehrzahl von Kapseln (14; 20, 22, 28) in mindestens einer äußeren Kapsel (12) aufgenommen ist und so eine innere Kapselungsstufe bildet und die Kapselwand (16) der äußeren Kapsel (12) ebenfalls ein umweltinduziertes Material umfaßt und die äußere Kapsel (12) eine äußere Kapselungsstufe bildet.

2. Mikrokapselsystem nach Anspruch 1, dadurch gekennzeichnet, daß mehr als zwei Kapselungsstufen vorhanden sind.

3. Mikrokapselsystem nach Anspruch 1 oder 2, dadurch gekennzeichnet, daß die Wände (24, 26) der Kapseln (20, 22) einer Kapselungsstufe gleiche umweltinduzierte Eigenschaften aufweisen.

4. Mikrokapselsystem nach Anspruch 1 oder 2, dadurch gekennzeichnet, daß die Wände (18, 30) der Kapseln (14, 28) einer Kapselungsstufe unterschiedliche umweltreaktive Eigenschaften aufweisen.

5. Mikrokapselsystem nach einem der vorhergehenden Ansprüche, dadurch gekennzeichnet, daß die Wände (16, 18; 24, 26, 30) der Kapseln (12, 14; 20, 22, 28) einer Kapselungsstufe andere umweltinduzierte Eigenschaften aufweisen als die von Kapseln (12, 14; 20, 22, 28) einer anderen Kapselungsstufe.

6. Mikrokapselsystem nach einem der vorhergehenden Ansprüche, dadurch gekennzeichnet, daß die Kapseln (12, 14; 20, 22, 28) einer Kapselungsstufe einen anderen Wirkstoff (W1, W2; A, B, C, D) enthalten als die Kapseln (12, 14; 20, 22, 28) einer anderen Kapselungsstufe.

7. Mikrokapselsystem nach einem der vorhergehenden Ansprüche, dadurch gekennzeichnet, daß Wände (14; 24, 26) von Kapseln (12; 20, 22) einer äußeren Kapselungsstufe zeitlich gesehen vor Wänden (18; 30) von Kapseln (14; 28) einer inneren Kapselungsstufe aufbrechen.

8. Mikrokapselsystem nach einem der Ansprüche 5 bis 7, dadurch gekennzeichnet, daß Wände (18) von Kapseln (14) einer inneren Kapselungsstufe zeitlich gesehen vor Wänden (16, 24) von Kapseln (12, 20) einer äußeren Kapselungsstufe aufbrechen.
